# EUROPÄISCHE PATENTSCHRIFT

(11) **EP 2 144 740 B1**
(45) Veröffentlichungstag und Bekanntmachung des Hinweises auf die Patenterteilung: **17.02.2016**
(21) Anmeldenummer: 08735200.1
(22) Anmeldetag: 12.04.2008
(51) Int. Cl.: A61M 5/32, B29C 45/16, B29L 31/00

(54) **VERFAHREN ZUR HERSTELLUNG EINES KUNSTSTOFFPRODUKTS MIT EINER KUNSTSTOFF-HARTKOMPONENTE UND EINER KUNSTSTOFF-WEICHKOMPONENTE SOWIE NACH DEM VERFAHREN HERGESTELLTES KUNSTSTOFFPRODUKT**
METHOD FOR THE PRODUCTION OF A PLASTIC PRODUCT HAVING A HARD PLASTIC COMPONENT AND A SOFT PLASTIC COMPONENT, AND PLASTIC PRODUCT PRODUCED USING SAID METHOD
PROCÉDÉ DE FABRICATION D'UN PRODUIT EN PLASTIQUE COMPRENANT UN COMPOSANT DUR EN PLASTIQUE ET UN COMPOSANT MOU EN PLASTIQUE ET PRODUIT EN PLASTIQUE FABRIQUÉ SUIVANT LEDIT PROCÉDÉ

(30) Priorität: 16.05.2007 DE 102007023129
(43) Veröffentlichungstag der Anmeldung: 20.01.2010
(73) Patentinhaber: RAUMEDIC AG, 95213 Münchberg (DE)
(72) Erfinder: JAKOB, Thomas, 95100 Selb (DE)
(74) Vertreter: Hofmann, Matthias
(86) Internationale Anmeldenummer: PCT/EP2008/002915
(87) Internationale Veröffentlichungsnummer: WO 2008/138442

(56) Entgegenhaltungen:
- EP-A- 0 397 977
- DE-A1- 1 491 743
- DE-U1- 29 602 173
- US-A1- 2004 017 051
- US-A1- 2006 089 602

## Beschreibung

Die Erfindung betrifft ein Verfahren zur Herstellung eines Kunststoffprodukts mit einer Kunststoff-Hartkomponente und einer Kunststoff-Weichkomponente. Ferner betrifft die Erfindung ein mit einem derartigen Verfahren hergestelltes Kunststoffprodukt.

Derartige Zwei-Komponenten-Kunststoffprodukte sind in vielfacher Ausführung aus dem Stand der Technik, der US 2004/0017051 A1, der DE 296 02 173 U1, der DE 14 91 743 A1, der EP 0 397 977 A und der US 2006/089602 A1, bekannt. Aus DE 100 41 812 A ist ein Steckerelement für eine elektronische Baueinheit mit einer Weichkomponente und einer Hartkomponente bekannt. Die Weichkomponente dient dabei oft als Dichtelement oder zur Verbesserung der Haptik eines Hartkomponenten-Grundkörpers des Kunststoffprodukts, zum Beispiel als Griffelement, beispielsweise bei einer Zahnbürste. Weiterhin sind Kunststoffprodukte bekannt, bei denen eine Hartkomponente nachträglich mit einer weichen Funktionskomponente zur Dichtung, Verbindung, Dämpfung oder zur Verbesserung der Haptik verbunden wird. So eine nachträgliche Verbindung ist in der Regel aufwändig.

Es ist daher eine Aufgabe der vorliegenden Erfindung, ein Kunststoffprodukt der eingangs genannten Art derart weiterzubilden, dass die Weichkomponente in ihrer Form und Anordnung flexibler an Funktionsanforderungen angepasst werden kann.

Diese Aufgabe ist erfindungsgemäß gelöst durch ein Herstellungsverfahren für das Kunststoffprodukt mit den im Anspruch 1 angegebenen Merkmalen.

Erfindungsgemäß wurde erkannt, dass es nicht zwingend ist, die Hartkomponente bei der Herstellung eines mehrkomponentigen Kunststoffprodukts zuerst auszuformen, sondern dass es überraschenderweise auch möglich ist, mit der Formung der Weichkomponente zu beginnen. Das Spritzgießen der Hartkomponente kann ohne Weiteres so gesteuert werden, dass die Weichkomponente nicht unerwünscht durch die eingespritzte Hartkomponente deformiert wird. Aufgrund der Reihenfolge Weichkomponente/Hartkomponente beim Spritzgießen ergeben sich neue Möglichkeiten für die Anordnung und Formgebung der Weichkomponente, ohne dass hierzu ein besonderer Aufwand bei der Formgestaltung der Formteile des Spritzwerkzeugs getrieben werden muss. Insbesondere durch die Gestaltung des erfindungsgemäßen Verfahrens derart, das das Spritzgießen der Hartkomponente und das Spritzgießen der Weichkomponente in ein und demselben Spritzwerkzeug erfolgt, wird das erfindungsgemäße Verfahren vereinfacht und verkürzt. Die Weichkomponente kann nun an Positionen innerhalb der Hartkomponente spritzgegossen werden, die bei umgekehrter Reihenfolge, also Hartkomponente/Weichkomponente, nicht zugänglich wären. Über die Grenzfläche haften die beiden Kunststoffkomponenten sicher aneinander an, so dass ein stabiles Verbund-Kunststoffprodukt resultiert, bei dem die beiden Kunststoffkomponenten ihre jeweiligen Funktionen erfüllen können. Die Hartkomponente verleiht dabei dem Kunststoffprodukt Festigkeit bzw. Steifigkeit. Die Hartkomponente kann zudem ggf. mit weiteren Komponenten oder im Rahmen einer automatisierten Montage eine Montagehilfe darstellen. Darüber hinaus wird vor dem Spritzgießen der Weichkomponente in das Spritzwerkzeug eine Einlegekomponente eingebracht. Die Einlegekomponente wird zumindest abschnittsweise und zumindest teilumfänglich von der Weichkomponente umspritzt. Bei einem derartigen Verfahren liegt nach dem Spritzgießen der Weichkomponente diese gut haftend an der Einlegekomponente an, so dass zum Beispiel auf ein Verkleben der Weichkomponente verzichtet werden kann.

Eine weitere Aufgabe der Erfindung ist es, ein Kunststoffprodukt anzugeben, bei dem die Vorteile des erfindungsgemäßen Verfahrens besonders gut zum Tragen kommen.

Diese Aufgabe ist erfindungsgemäß gelöst durch ein in ein und demselben Spritzwerkzeug gefertigten Kunststoffprodukt nach einem der Ansprüche 2 oder 3.

Bei einem derartigen Kunststoffprodukt kommen die Vorteile der erfindungsgemäßen Spritzguss-Reihenfolge gut zum Tragen, da insbesondere auf zusätzliche Klebematerialien verzichtet werden kann, so dass die Herstellung eines medizinisch unbedenklichen Kunststoffprodukts vereinfacht ist.

Wenn gemäß Anspruch 2 die Weichkomponente von der Hartkomponente in Umfangsrichtung um eine Hauptentformungsrichtung des Spritzwerkzeugs umgeben ist, ist es durch die Spritzguss-Reihenfolge Weichkomponente/Hartkomponente möglich, beide Komponenten trotzdem durch Spritzguss herzustellen. Die Einlegekomponente ist von der Weichkomponente zumindest abschnittsweise und zumindest teilumfänglich umspritzt.

Ein Härtebereich der Weichkomponente nach Anspruch 3 ermöglicht insbesondere eine Weichkomponente mit guter Dichtfunktion. Dabei haftet die Kunststoff-Weichkomponente an der Kunststoff-Hartkomponente über mindestens eine Grenzfläche an, wobei die Weichkomponente von der Hartkomponente in Umfangsrichtung um eine Hauptentformungsrichtung eines Spritzwerkzeugs zumindest teilumfänglich umgeben ist. Ein derartiges medizinisches Arbeitsmittel weist die Kunststoff-Weichkomponente und die Kunststoff-Hartkomponente auf. Insbesondere eine Einlegekomponente ist bei diesem Arbeitsmittel nicht zwingend erforderlich.

Materialien nach den Ansprüchen 4 bis 7 haben sich zur Herstellung eines erfindungsgemäßen und zwei Komponenten aufweisenden Kunststoffprodukts als besonders geeignet herausgestellt. Anstelle eines thermoplastischen Elastomers kann als Weichkomponente auch ein Silikon eingesetzt werden. Ein thermoplastisches Elastomer nach Anspruch 3 und ein Thermoplast nach Anspruch 4 haften gut aneinander an. Neben TPE-V und TPE-S können noch die folgenden thermoplastischen Elastomere für die Weichkomponente eingesetzt werden: TPE-A (Amid-Basis), TPE-U (Urethan-Basis), TPE-E (Ether- bzw. Ester-Basis) und TPE-O (Olefin-Basis). Als Styrol-Basis können insbesondere SBS (Styrol-Butadien-Styrol-BlockCopolymere), SEBS (Styrol-Ethylen-Butadien-Styrol-Copolymere) und SIS (Styrol-Isopren-Styrol-Copolymere) eingesetzt werden. Aufgrund der guten Haftung werden als bevorzugte Materialkombination insbesondere Polypropylene in Form von Homo-, Block- oder Random-Polypropylene als Hartkomponente und thermoplastische Elastomere auf Styrol-Basis, Olefin-Basis sowie vernetzte thermoplastische Elastomere als Weichkomponente eingesetzt. Derartige Materialkombinationen ergeben eine gute Verbundfestigkeit und lassen sich zudem wirtschaftlich herstellen.

Bei einer Injektionsnadel nach Anspruch 9, die zum Beispiel als Luer-Nadel ausgeführt sein kann, sorgt die Weichkomponente für eine sichere Abdichtung zwischen einer als Einlegekomponente ausgeführten Injektionskanüle und der den Grundkörper vorgebenden Hartkomponente.

Ein Ausführungsbeispiel der Erfindung wird nachfolgend anhand der Zeichnung näher erläutert. In dieser zeigen:
- Fig. 1: eine perspektivische Ansicht einer Luer-Nadel als Beispiel für ein erfindungsgemäß hergestelltes Kunststoffprodukt; und
- Fig. 2: einen Längsschnitt durch die Luer-Nadel nach Fig. 1.

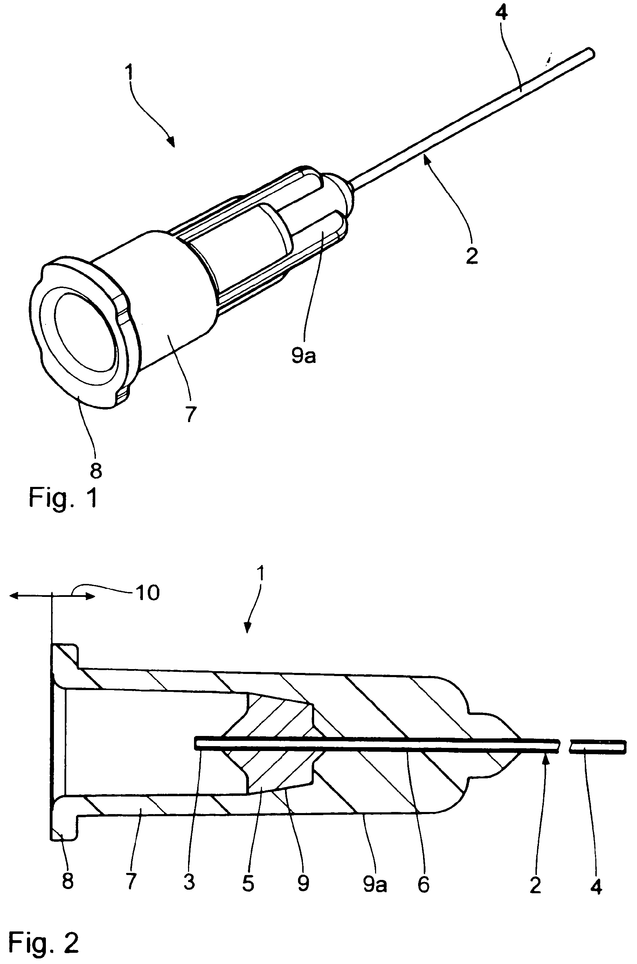

Eine insgesamt mit 1 bezeichnete Luer-Nadel ist ein Beispiel für ein erfindungsgemäß hergestelltes Kunststoffprodukt. Die Luer-Nadel hat als Injektionskanüle eine Stahlnadel 2. In einem in der Fig. 2 linken Endbereich 3 der Stahlnadel 2, der einem freien Endbereich 4 der Stahlnadel 2 gegenüberliegt, ist die Stahlnadel 2 vollumfänglich von einer Kunststoff-Weichkomponente 5 umgeben. Bei der Kunststoff-Weichkomponente 5 handelt es sich um TPE-V, also um ein vernetztes thermoplastisches Elastomer. Letzteres hat eine Shore-Härte zwischen A30 und A70.

Die Kunststoff-Weichkomponente 5, der Endbereich 3 sowie ein sich hieran in der Fig. 2 rechts anschließender Mittelbereich 6 der Stahlnadel 2 sind voll umfänglich umschlossen von einer Kunststoff-Hartkomponente 7. Letztere bildet den Kunststoff-Grundkörper der Luer-Nadel 1.

Die Kunststoff-Hartkomponente 7 ist äußerlich geformt, wie dies bei Luer-Nadeln an sich bekannt ist, hat also einen üblichen Verschlusskragen 8 sowie insgesamt vier Angriffsflügel 9a, die eine Drehbewegung der Luer-Nadel 1 um die Längsachse der Stahlnadel 2 zur Herstellung einer Verbindung der Luer-Nadel 1 mit einem Spritzenbehälter erleichtern. Der Verschlusskragen 8 dient dabei als Kunststoff-Anschlusselement zur Verbindung der Luer-Nadel 1 mit dem Spritzenbehälter. Die Kunststoff-Hartkomponente ist aus Polypropylen, kann aber auch aus einem anderen Thermoplasten gefertigt sein.

Über eine um die Längsachse der Stahlnadel 2 rotationssymmetrische Grenzfläche 9 haftet die Kunststoff-Hartkomponente 7 an der Kunststoff-Weichkomponente 5 an. Die Kunststoff-Weichkomponente 5 dient zur Abdichtung der Stahlnadel 2 gegen die Kunststoff-Hartkomponente 7.

Die Luer-Nadel 1 wird folgendermaßen hergestellt:
Zunächst wird die Stahlnadel 2 in ein nicht dargestelltes Spritzwerkzeug als Einlegeteil eingelegt. Anschließend wird die Kunststoff-Weichkomponente 5 in dem Spritzgusswerkzeug spritzgegossen. Die plastifizierte Weichkomponente wird im Spritzgusswerkzeug dabei über einen Tunnelanguss zugeführt. Nach dem Einspritzen und Aushärten der Weichkomponente 5 werden Werkzeughälften, die die Form der Kunststoff-Weichkomponente 5 begrenzen, längs einer Hauptentformungsrichtung 10 getrennt. In Bezug auf diese Hauptentformungsrichtung 10 ist die Kunststoff-Weichkomponente 5 hinterschneidungsfrei.

Im Anschluss an das Spritzgießen der Kunststoff-Weichkomponente 5 wird die Kunststoff-Hartkomponente 7 spritzgegossen. Die plastifizierte Hartkomponente wird im Spritzgusswerkzeug dabei über einen weiteren Tunnelanguss zugeführt. Das Spritzgießen der Hartkomponente 7 erfolgt erst, wenn die Weichkomponente 5 so weit ausgehärtet ist, dass eine unerwünschte Deformation der Weichkomponente 5 durch die eingespritzte Hartkomponente 7 vermieden ist. Die Hartkomponente 7 umspritzt dabei abschnittsweise die Stahlnadel 2 sowie die Kunststoff-Weichkomponente 5.

Nach dem Einspritzen und Aushärten der Hartkomponente 7 werden Spritzgusswerkzeughälften, die die Form der Kunststoff-Hartkomponente 7 begrenzen, ebenfalls längs der Hauptentformungsrichtung 10 voneinander getrennt. Längs der Hauptentformungsrichtung 10 ist auch die Hartkomponente 7 hinterschneidungsfrei.

Die an die Weichkomponente 5 über die Grenzfläche 9 angespritzte Hartkomponente 7 haftet adhäsiv an der Weichkomponente 5. Zudem haften sowohl die Weichkomponente 5 als auch die Hartkomponente 7 adhäsiv an der Stahlnadel 2. Der Einsatz eines zusätzlichen Klebemittels entfällt.

Die Weichkomponente 5 ist in Umfangsrichtung um die Hauptentformungsrichtung 10, die mit der Längsachse der Stahlnadel 2 zusammenfällt, voll umfänglich von der Hartkomponente 7 umgeben.

## Patentansprüche

1. Verfahren zur Herstellung eines medizinischen Arbeitsmittels (1), insbesondere einer Injektionsnadel, mit einer Kunststoff-Hartkomponente (7) und einer Kunststoff-Weichkomponente (5), die an der Kunststoff-Hartkomponente (7) über mindestens eine Grenzfläche (9) anhaftet, mit folgenden Schritten:
- Spritzgießen der Weichkomponente (5) in einem Spritzwerkzeug,
- Spritzgießen der Hartkomponente (7) in dem Spritzwerkzeug im Anschluss an das Spritzgießen der Weichkomponente (5), wobei vor dem Spritzgießen der Weichkomponente (5) in das Spritzwerkzeug eine Einlegekomponente (2) eingebracht wird, die von der Weichkomponente (5) zumindest abschnittsweise und zumindest teilumfänglich umspritzt wird.

2. Medizinisches Arbeitsmittel (1) als stabiles Verbund-Kunststoffprodukt, insbesondere eine Injektionsnadel, mit einer Kunststoff-Hartkomponente (7), mit einer Kunststoff-Weichkomponente (5), die an der Kunststoff-Hartkomponente (7) über mindestens eine Grenzfläche (9) anhaftet, wobei die Weichkomponente (5) von der Hartkomponente (7) in Umfangsrichtung um eine Hauptentformungsrichtung (10) des Spritzwerkzeugs zumindest teilumfänglich umgeben ist, hergestellt in dem Spritzwerkzeug nach einem Verfahren nach Anspruch 1, wobei eine Einlegekomponente (2) von der Weichkomponente (5) zumindest abschnittsweise und zumindest teilumfänglich umspritzt ist.

3. Medizinisches Arbeitsmittel nach Anspruch 2
**dadurch gekennzeichnet, dass**
- die Weichkomponente (5) aus einem thermoplastischen Elastomer (TPE) gefertigt ist und
- das thermoplastische Elastomer eine Shore-Härte im Bereich von A5 bis D70, insbesondere im Bereich von A30 bis A70, aufweist.

4. Medizinisches Arbeitsmittel nach einem der Ansprüche 2 oder 3, **dadurch gekennzeichnet, dass** die Hartkomponente (7) aus einem Thermoplasten gefertigt ist.

5. Medizinisches Arbeitsmittel nach einem der Ansprüche 2 bis 4, **dadurch gekennzeichnet, dass** die Hartkomponente (7) aus Polypropylen gefertigt ist.

6. Medizinisches Arbeitsmittel nach einem der Ansprüche 2 bis 5, **dadurch gekennzeichnet, dass** die Weichkomponente (5) aus vernetztem TPE (TPE-V) gefertigt ist.

7. Medizinisches Arbeitsmittel nach einem der Ansprüche 2 bis 5, **dadurch gekennzeichnet, dass** die Weichkomponente (5) aus einem thermoplastischen Elastomer auf Styrolbasis (TPE-S) gefertigt ist.

8. Medizinisches Arbeitsmittel nach einem der Ansprüche 2 bis 7, **gekennzeichnet durch** seine Ausgestaltung als Injektionsnadel (1) mit einem Kunststoff-Anschlusselement (8) an der Kunststoff-Hartkomponente zur Verbindung mit einem Spritzenbehälter.

## Claims

1. A method for producing a medical implement (1), in particular an injection needle, having a hard plastic component (7) and a soft plastic component (5) that adheres to the hard plastic component (7) via at least one boundary surface (9), comprising the following steps:
- injection molding of the soft component (5) in an injection molding die,
- injection molding of the hard component (7) following the injection molding of the soft component (5), wherein prior to the injection molding of the soft component (5) an insert component (2) is inserted into the injection molding die that is coated by injection molding at least in some sections thereof and over at least part of the circumference thereof with the soft component (5).

2. A medical implement as a sturdy composite plastic product (1), in particular an injection needle, having a hard plastic component (7) and a soft plastic component (5) that adheres to the hard plastic component (7) via at least one boundary surface (9), wherein the soft component (5) is surrounded over at least part of the circumference thereof by the hard component (7) in the circumferential direction about a main demolding direction (10) of the injection molding die, produced in the injection molding die according to a method according to claim 1, wherein an insert component (2) is coated by injection molding at least in some sections thereof and at least part of the circumference thereof with the soft component (5).

3. A medical implement according to claim 2,
**characterized in that**
- the soft component (5) is produced of a thermoplastic elastomer (TPE) and
- the thermoplastic elastomer has a shore hardness in the range of A5 to D70, especially in the range of A30 to A70.

4. A medical implement according to one of claims 2 or 3, **characterized in that** the hard component (7) is produced of a thermoplastic.

5. A medical implement according to one of claims 2 to 4, **characterized in that** the hard component (7) is produced of polypropylene.

6. A medical implement according to one of claims 2 to 5, **characterized in that** the soft component (5) is produced of cross-linked TPE (TPE-V).

7. A medical implement according to one of claims 2 to 5, **characterized in that** the soft component (5) is produced of a thermoplastic elastomer on styrene basis (TPE-S).

8. A medical implement according to one of claims 2 to 7, **characterized by** its design in the form of an injection needle (1) having a plastic connecting element (8) on the hard plastic component for connecting to a syringe container.

## Revendications

1. Procédé servant à fabriquer un instrument de travail (1) médical, en particulier une aiguille d'injection, comprenant un composant dur en plastique (7) et un composant mou en plastique (5), qui adhère au composant dur en plastique (7) par l'intermédiaire au moins d'une interface (9),
comprenant les étapes suivantes consistant à :
- mouler par injection le composant mou (5) dans un outil de pulvérisation,
- mouler par injection le composant dur (7) dans l'outil de pulvérisation immédiatement après le moulage par injection du composant mou (5), sachant qu'est introduit, avant le moulage par injection du composant mou (5) dans l'outil de pulvérisation, un composant d'insertion (2), qui est enrobé par injection au moins par endroits et au moins de manière partiellement périphérique par le composant mou (5).

2. Instrument de travail (1) médical sous la forme d'un produit en plastique composite stable, en particulier une aiguille d'injection, comprenant un composant dur en plastique (7), un composant mou en plastique (5), qui adhère au composant dur en plastique (7) par l'intermédiaire au moins d'une interface (9), sachant que le composant mou (5) est entouré au moins de manière partiellement périphérique par le composant dur (7) dans la direction périphérique autour d'une direction de déformation principale (10) de l'outil de pulvérisation, fabriqué dans l'outil de pulvérisation selon un procédé selon la revendication 1, sachant qu'un composant d'insertion (2) est enrobé par pulvérisation du composant mou (5) au moins par endroits et au moins de manière partiellement périphérique.

3. Instrument de travail selon la revendication 2, **caractérisé en ce**
- **que** le composant mou (5) est confectionné à partir d'un élastomère thermoplastique (TPE), et
- en ce que l'élastomère thermoplastique présente une dureté Shore comprise dans la plage allant de A5 à D70, en particulier comprise dans la plage allant de A30 à A70.

4. Instrument de travail médical selon l'une quelconque des revendications 2 ou 3, **caractérisé en ce que** le composant dur (7) est confectionné à partir d'une matière thermoplastique.

5. Instrument de travail médical selon l'une quelconque des revendications 2 à 4, **caractérisé en ce que** le composant dur (7) est confectionné à partir d'un polypropylène.

6. Instrument de travail médical selon l'une quelconque des revendications 2 à 5, **caractérisé en ce que** le composant mou (5) est confectionné à partir d'un TPE réticulé (TPE-R).

7. Instrument de travail médical selon l'une .05 des revendications 2 à 5, **caractérisé en ce que** le composant mou (5) est confectionné à partir d'un élastomère thermoplastique à base de styrol (TPE-S).

8. Instrument de travail médical selon l'une quelconque des revendications 2 à 7, **caractérisé par** sa configuration sous la forme d'une aiguille d'injection (1) comprenant un élément de raccordement en plastique (8) au composant dur en plastique destiné à être relié à un bac à seringues.
